# EUROPEAN PATENT APPLICATION

(11) **EP 4 809 993 A1**
(43) Date of publication of application: **23.09.2026**
(21) Application number: 25165473.7
(22) Date of filing: 21.03.2025
(51) Int. Cl.: A61F 13/494, A61F 13/495

(54) **ABSORBENT ARTICLE WITH GUARD POCKET WITH HYDROPHILIC SURFACE**

(71) Applicant: Drylock Technologies NV, 9240 Zele (BE)
(72) Inventor: DERYCKE, Tom, 9240 Zele (BE); SMET Steven, 9240 Zele (BE)
(74) Representative: Arnold & Siedsma

(57) **Abstract**

An absorbent articles, such as diapers, are described herein, in particular the articles as described herein have guard pockets.

## Description

### TECHNICAL FIELD

The present invention pertains to the technical field of absorbent articles, more preferably disposable personal care articles such as diapers, baby pants, adult incontinent garments, and the like. More specifically the present invention relates to an absorbent article, preferably of the pants-type, comprising an elastic front section, an elastic rear section, and an absorbent body extending in a longitudinal direction between the elastic front section and the elastic rear section.

### BACKGROUND

Diapers when being used may contain loose fecal matter, especially when used by infants and toddlers experiencing loose stools or during periods of high activity. This can lead to soiling of clothing and bedding, requiring frequent changes and cleanups, which may pose a burden on caregivers.

To address this issue, some diaper designs have explored the concept of "guard pockets" or similar structures integrated into at or near the waist region. These pockets aim to create a physical barrier that traps and contain the loose fecal matter, preventing it from escaping the diaper.

However, there remains a need for an improved design incorporating guard pockets which can further improve comfort of a wearer and preferably the comfort of caregivers.

### SUMMARY

Hence, it is an object of embodiments herein to provide an absorbent article, with a good protection against the escape of bodily exudates at the waistline while aiming for a good fit and providing comfort for a wearer. More in particular, there is an aim to further improve comfort for a wearer.

According to aspects of the invention, there is provided an absorbent article comprising an elastic front section, an elastic rear section, and an absorbent body extending in a longitudinal direction between the elastic front section and the elastic rear section. The elastic front section and the elastic rear section each comprise a laminate comprising, preferably consisting of an inner sheet, an outer sheet, and at least one elastic member positioned between the inner sheet and the outer sheet. The absorbent body comprises a liquid pervious topsheet, a liquid impervious backsheet, and an absorbent core positioned between the topsheet and backsheet.

The absorbent article comprising:
- a rear guard pocket extending transversely across the elastic rear section, the rear guard pocket having a rear guard pocket opening for receiving excretions (such as poo) the rear guard pocket comprises a hydrophilic surface (Aₚₕᵢ); and/or
- a front guard pocket extending transversely across the elastic front section, the front guard pocket having a front guard pocket opening for receiving excretions (such as pee) the front guard pocket comprises a hydrophilic surface (Aₚₕᵢ).

A further aspect provides for an absorbent body extending in a longitudinal direction between a front section and a rear section of the waistband portion,
wherein the article comprises fastening means, such as a pair of fastening tabs, for fastening the rear section of the waistband portion to the front section the waistband portion,
wherein the absorbent body comprises a liquid pervious topsheet, a liquid impervious backsheet, and an absorbent core positioned between the topsheet and backsheet; the absorbent article comprising
at least one waist guard pocket (such as a front and/or a rear guard pocket), extending transversely across the waistband portion, the waist guard pocket having a pocket opening for receiving excretions; wherein the waist guard pocket comprises a hydrophilic surface (Aₚₕᵢ). Surprisingly, comfort for a wearer and potential caregivers could be enhanced by ensuring a hydrophilic surface at the location of the pocket, in particular due to a result of an improved capturing of excretions.

Namely, inventive insights have provided that by using a hydrophilic surface (as compared to hydrophobic), bodily exudates may be better contained in or by the pocket when the hydrophilic surface. The better containment within the pocket further improves comfort of the wearer. As a result, and advantageously, leakage prevention may be further improved.

Additionally, inventive insights have provided that by using a hydrophilic surface (as compared to hydrophobic) at the outside of the pocket, that comfort of the wearer can be improved. Namely, the hydrophilic surface may increase the softness which enhances comfort (e.g. softness felt by the belly when worn and/or softness felt back the lower back, such comfort may be further increase when both a front guard and back guard pocket are included, also called Pee & Poo Pocket). Additionally, the hydrophilic surface may better capture vapour/damp from the skin which further increases the feel of extra softness (e.g. on the back and/or belly area of a wearer), as a result comfort is further improved.

Because bodily exudates typically include a great share of water, they may better "stick" to the hydrophilic surface. Accordingly, a hydrophilic surface, e.g. provided by a nonwoven with a hydrophilic spin finish, can better contain the bodily exudates.

According to a preferred embodiment, the articles comprises the front guard pocket and the rear guard pocket. By comprises the front guard pocket and the rear guard pocket, the containment of excretions is improved significantly.

The elastic rear section preferably comprises a rear folded portion wherein the laminate of the elastic rear section is folded from a rear waistline of the elastic rear section towards the topsheet of the absorbent body. The rear folded portion extends from the rear waistline to a free edge of the rear folded portion to form the rear guard pocket wherein the rear pocket opening is defined between the free edge of the rear folded portion and the topsheet of the absorbent body. By providing a fold from the rear waistline of the elastic rear section towards the topsheet of the absorbent body an additional wall is created, in the form of the rear folded portion, to prevent bodily exudates to leak out of the absorbent article at the rear waistline thereof. When such bodily exudates would move from the absorbent body towards the rear waistline, they will enter the provided rear guard pocket via the pocket opening. The rear guard pocket, and more in particular the rear folded section will then act as a barrier which prevents the bodily exudates to move or migrate beyond the rear waistline. Moreover, by folding the laminate, i.e. folding both the inner sheet and outer sheet, the folded portion has an improved integrity, both in dry state and wet state. In addition, the folded laminate provides additional support and sturdiness in a waistline area of the absorbent article thereby increasing comfort for a wearer. In addition, it should be noted that no separate layers or sheets of material are required to provide the guard pocket, which renders the absorbent article to be manufactured in a more efficient and economic manner as compared to known absorbent articles having a guard pocket.

It may also be possible, that the front and/or rear guard pocket is provided as a patch, wherein a perimeter of the patch is sealed to the respective elastic section (rear or front).

Preferably the rear folded portion comprises a first elastic member in proximity of and substantially parallel with the free edge, and a first attachment zone wherein the inner sheet and outer sheet are attached to each other, wherein the first attachment zone is positioned in between the free edge and the first elastic member.

By providing an elastic member and a corresponding attachment zone in proximity of the free edge of the rear folded portion, a structural integrity of the free edge is further improved. The provision and position of the elastic member cause the guard pocket to advantageously open up when the absorbent article is worn around the waist of a wearer. In addition, the attachment zone makes sure that the elastic member remains in place and that the laminate remains intact.

Preferably a maximum distance between the first elastic member and the free edge is less than 15mm, more preferably less than 12mm, even more preferably less than 9mm, and most preferably less than 6mm.

It has been found that the first elastic member should be positioned as close as possible to the free edge to yield the best results in terms of structural integrity and opening up of the guard pocket during use.

Preferably the rear folded portion comprises a second attachment zone wherein the inner sheet and outer sheet are attached to each other, and wherein the second attachment zone is positioned in between the first elastic member and the rear waistline.

Preferably the rear folded portion comprises at least one second elastic member positioned between the first elastic member and the second attachment zone.

Preferably the rear folded portion comprises a closed edge portion wherein the inner sheet of the rear folded portion is attached to the inner sheet of the elastic rear portion, wherein the closed edge portion extends from the rear waistline in the direction of the pocket opening.

Preferably the inner sheet comprises a spunbond nonwoven and/or meltblown nonwoven, which may be treated to include hydrophilic surface. In a preferred embodiment the inner sheet is a spunbond-meltblown-spunbond nonwoven. Preferably the inner sheet has a basis weight of between 5-20 gsm, more preferably 6-17 gsm, even more preferably 7-14 gsm, and most preferably 8-12 gsm. These materials have the benefit of being hydrophobic and thereby add to the liquid-impermeability of the rear folded portion and rear guard pocket. In addition, the presence of a meltblown component enhances the ability of the inner sheet to be thermosealed in an efficient manner. To even further improve the inner sheet, a hydrophobic finish or treatment may be applied.

Preferably the outer sheet comprises a spunbond and/or trough air bonded nonwoven, which may be treated to include hydrophilic surface. In a preferred embodiment the outer sheet is a spunbond and/or trough air bonded nonwoven. Preferably the outer sheet has a basis weight of between 8-25 gsm, more preferably 9-20 gsm, even more preferably 10-15 gsm. These materials have the benefit of being soft to the touch. By providing a rear folded portion, the outer sheet of the rear folded portion will be in contact with the skin of a wearer thereby improving comfort.

Preferably, at least one preferably both the inner sheet and outer sheet are treated to have hydrophilic surface. In this manner, comfort can be improved.

Preferably the elastic front section comprises a front folded portion wherein the laminate of the elastic front section is folded from a front waistline of the elastic front section towards the topsheet of the absorbent body; and wherein the front folded portion extends from the front waistline to a free edge of the front folded portion to form a front guard pocket having a pocket opening defined between the free edge of the front folded portion and the topsheet of the absorbent body.

By providing a fold from the front waistline of the elastic rear section towards the topsheet of the absorbent body an additional wall is created, in the form of the front folded portion, to prevent bodily exudates to leak out of the absorbent article at the front waistline thereof. When such bodily exudates would move from the absorbent body towards the front waistline, they will enter the provided front guard pocket via the pocket opening. When entered and contacted with hydrophilic surface, improved capture can be achieved.

The front guard pocket, and more in particular the front folded section will then act as a barrier which prevents the bodily exudates to move or migrate beyond the front waistline, while the hydrophilic surface acts to capture or absorb the exudates. Moreover, by folding the laminate, i.e. folding both the inner sheet and outer sheet, the front folded portion has an improved integrity, both in dry state and wet state. In addition, the folded laminate provides additional support and sturdiness in a waistline area of the absorbent article thereby increasing comfort for a wearer.

Preferably the front folded portion comprises a first elastic member in proximity of and substantially parallel with the free edge, and a first attachment zone wherein the inner sheet and outer sheet are attached to each other wherein the first attachment zone is positioned in between the free edge and the first elastic member, preferably wherein at least one of the inner sheet and outer sheet comprises hydrophilic surface.

Preferably a maximum distance between the first elastic member and the free edge of the front folded portion is less than 15mm, more preferably less than 12mm, even more preferably less than 9mm, and most preferably less than 6mm.

According to a further aspect of the invention, there is provided an absorbent article comprising an elastic front section, an elastic rear section, and an absorbent body extending in a longitudinal direction between the elastic front section and the elastic rear section. The elastic front section and the elastic rear section each comprise a laminate comprising, preferably consisting of an inner sheet, an outer sheet, and at least one elastic member positioned between the inner sheet and the outer sheet. The absorbent body comprises a liquid pervious topsheet, a liquid impervious backsheet, and an absorbent core positioned between the topsheet and backsheet. The elastic front section comprises a front folded portion wherein the laminate of the elastic front section is folded from a front waistline of the elastic front section towards the topsheet of the absorbent body. The front folded portion extends from the front waist line to a free edge of the front folded portion to form a front guard pocket having a pocket opening defined between the free edge of the front folded portion and the topsheet of the absorbent body.

By providing a fold from the front waistline of the elastic rear section towards the topsheet of the absorbent body an additional wall is created, in the form of the front folded portion, to prevent bodily exudates to leak out of the absorbent article at the front waistline thereof. When such bodily exudates would move from the absorbent body towards the front waistline, they will enter the provided front guard pocket via the pocket opening, the hydrophilic surface may enhance the pocket performance due to improved interaction with the bodily exudates. In particular in cases where the pocket overlaps with the absorbent core as seen from a top view. Namely, in such as case the pocket preferably has an internal surface which is made hydrophilic to improve dissipation of the materials. The front guard pocket, and more in particular the front folded section will then act as a barrier which prevents the bodily exudates to move or migrate beyond the front waistline. Moreover, by folding the laminate, i.e. folding both the inner sheet and outer sheet, the front folded portion has an improved integrity, both in dry state and wet state. In addition, the folded laminate provides additional support and sturdiness in a waistline area of the absorbent article thereby increasing comfort for a wearer.

Preferably the front folded portion comprises a first elastic member in proximity of and substantially parallel with the free edge, and a first attachment zone wherein the inner sheet and outer sheet are attached to each other wherein the first attachment zone is positioned in between the free edge and the first elastic member.

Preferably a maximum distance between the first elastic member and the free edge is less than 15mm, preferably less than 12mm, more preferably less than 9mm, and most preferably less than 6mm. Preferably the front folded portion comprises a second attachment zone wherein the inner sheet and outer sheet are attached to each other, and wherein the second attachment zone is positioned in between the first elastic member and the front waistline. Preferably wherein the area between the first and second attachment zone is made hydrophilic. Preferably the front folded portion comprises at least one second elastic member positioned between the first elastic member and the second attachment zone. Preferably the front folded portion comprises a closed edge portion wherein the inner sheet of the front folded portion is attached to the inner sheet of the elastic front portion, wherein the closed edge portion extends from the front waistline in the direction of the pocket opening.

Preferably the inner sheet (of the front and/or rear and/or guard pocket) comprises a spunbond nonwoven and/or meltblown nonwoven. In a preferred embodiment the inner sheet is a spunbond-meltblown-spunbond nonwoven. Preferably the inner sheet has a basis weight of between 5-20 gsm, more preferably 6-17 gsm, even more preferably 7-14 gsm, and most preferably 8-12 gsm. Preferably, said inner sheet has been treated with a hydrophilic finish. The finish can provide a durable hydrophilic performance.

Preferably the outer sheet (of the front and/or rear and/or guard pocket) comprises a spunbond and/or trough air bonded nonwoven. In a preferred embodiment the outer sheet is a spunbond and/or trough air bonded nonwoven. Preferably the outer sheet has a basis weight of between 8-25 gsm, more preferably 9-20 gsm, even more preferably 10-15 gsm. Preferably, said outer sheet has been treated with a hydrophilic finish. The finish can provide a durable hydrophilic performance.

Preferably the absorbent core comprises absorbent material and at least one channel, wherein less absorbent material per surface area is present in the at least one channel as compared to an area around the at least one channel, wherein preferably substantially no absorbent material is present in the at least one channel.

Preferably the at least one channel extends substantially in the longitudinal direction.

Preferably a bottom core wrap of the absorbent core is attached to a top core wrap of the absorbent core at the at least one channel.

Additional features and benefits which are described above and below in view of embodiments having a rear folded portion according to the first aspect apply similarly to embodiments having a front rear folded portion according to the second aspect, mutatis mutandis, and vice versa.

A further aspect provides for an absorbent article, such as a diaper which can be refastened, comprising an absorbent body extending in a longitudinal direction between a front section and a rear section of a waistband portion, wherein the article comprises fastening means, such as a pair of fastening tabs, for fastening the rear section a waistband portion to the front section the waistband portion, wherein the absorbent body comprises a liquid pervious topsheet, a liquid impervious backsheet, and an absorbent core positioned between the topsheet and backsheet; the absorbent article comprising
a waist guard pocket, such as a front or rear guard pocket, extending transversely across the waistband portion, the waist guard pocket having a pocket opening for receiving excretions, such as urine or feces; wherein the waist guard pocket comprises a hydrophilic surface (Aphi). The hydrophilic surface of the waist guard pocket can enhance comfort as described above.

As applicable to the pockets as described herein the context of all aspects (related to front and/or rear guard and/or waist guard pocket), the following preferred features shall now be explained.

Preferably, the guard pocket (including reference to the front and/or rear and/or the waist guard pockets) comprises an inside portion and an outside portion.

The "inside portion" of the pocket is understood as the portion enclosed by the respective pocket. To be faced towards the inside of the pocket or inside portion of the pocket can be used interchangeably herein.

The "outside portion" of the pocket is understood as the outside portion to be faced towards a wearer when worn. To be faced towards the outside of the pocket or outside portion of the pocket can be used interchangeably herein.

The hydrophilic surface (Aₚₕᵢ) may be provided to be present on the inside portion and/or on the outside portion the front and/or rear guard pocket.

According to an embodiment, the hydrophilic surface (Aₚₕᵢ) is present on the inside portion of the front guard pocket. Accordingly, excretions (such as pee) may be better contained within the pocket, consequently comfort may be further improved.

According to an embodiment, hydrophilic surface (Aₚₕᵢ) is present on the outside portion of the front guard pocket. The hydrophilic surface on the outside portion of the front guard pocket may better capture vapour/damp from the skin which further increases the feel of extra softness (e.g. belly area of a wearer).

According to an embodiment, hydrophilic surface (Aₚₕᵢ) is present on the outside portion of the rear guard pocket. The hydrophilic surface on the outside portion of the rear guard pocket may better capture vapour/damp from the skin which further increases the feel of extra softness (e.g. lower back area of a wearer).

According to an embodiment, hydrophilic surface (Aₚₕᵢ) is present on the inside portion of the rear guard pocket. Accordingly, excretions (such as poo) may be better contained within the pocket, consequently comfort may be further improved. Furthermore, comfort of a caregiver can be further improved since excretions are better contained, which facilitates potential burdens during care (e.g. diaper change).

The hydrophilic surface (Aₚₕᵢ) is preferably present as surface of a nonwoven, preferably a nonwoven chosen from the group consisting of Spunbond nonwoven, Spunlace nonwoven, Meltblown nonwoven or combinations thereof.

According to an embodiment, the hydrophilic surface (Aphi) is present as a surface of a nonwoven chosen from a treated nonwoven. The treated nonwoven may be a treated nonwoven comprising polyethylene (PE), polypropylene (PP), polyester or polyethylene terephthalate (PET) or combinations thereof. The treated nonwoven may be treated with a hydrophilic coating or a hydrophilic finish, preferably hydrophilic finish.

According to an embodiment, hydrophilic surface (Aphi) is a surface of a hydrophilic nonwoven chosen from a nonwoven which substantially consist of a hydrophilic material(s).

According to an embodiment, the hydrophilic surface (Aphi) is a surface of a hydrophilic nonwoven chosen from a nonwoven comprising a mixture of hydrophobic polymers blended or grafted with hydrophilic additives (also called hydrophilic agents).

Preferably, the hydrophilic surface (Aphi) is provided by a surface coating, such as a surfactant coating, a plasma treatment, a corona discharge treatment.

Preferably, the hydrophilic surface (Aphi) is provided by a surfactant coating on the inside portion of the pocket, preferably to lower the contact angle between and liquids like urine, promoting rapid absorption and distribution within the pocket (front and/or rear).

Preferably, the hydrophilic surface (Aphi) is provided by aid of a hydrophilic surface coating or by aid of hydrophilic finish.

Preferably, the hydrophilic surface (Aphi) is provided by aid of hydrophilic spin finish. The hydrophilic spin finish can enhance the durability of the hydrophilic surface.

Preferably, the pocket comprises a spunbond nonwovens comprising polypropylene which is treated with a spin finish.

Preferably, the hydrophilic surface (Aₚₕᵢ) is provided by a hydrophilic nonwoven chosen from the group consisting of: a nonwoven treated with a hydrophilic coating or hydrophilic finish or both; and/or
a nonwoven obtained by mixing a hydrophilic agent directly in a thermoplastic polymer resin before being processed into the nonwoven , e.g. via spunbonding, melt blowing, dry-laying, wet-laying, spunlacing or hydroentanglement

According to an embodiment, the hydrophilic surface (Aₚₕᵢ) is present as a surface of a nonwoven arranged as an inner sheet of the pocket; and/or wherein the hydrophilic surface (Aₚₕᵢ) is present as a surface of a nonwoven arranged as an outer sheet of the pocket.

### BRIEF DESCRIPTION OF FIGURES

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figures 1A and 1B are schematic top plan views of an exemplary embodiment of an absorbent article according to the first aspect of the invention, wherein the elastic rear section is illustrated in its flat-out and folded state, respectively;
Figures 1C and 1D are schematic side views in the transversal direction of the elastic rear section of the exemplary embodiment of Figure 1A and Figure 1B, wherein the elastic rear section is illustrated in its flat-out and folded state, respectively; Figures 1E, Figure 1F, Figure 1G are schematic side views in the transversal direction of the elastic rear section showing several options for where the hydrophilic surface (Aₚₕᵢ) can be present.
Figures 2A and Figure 2B are schematic top plan views of an exemplary embodiment of an absorbent article according to the second aspect of the invention, wherein the elastic front section is illustrated in its flat-out and folded state, respectively.
Figure 2C and Figure 2D are schematic side views in the transversal direction of the elastic front section of the exemplary embodiment of figures 2A and Figure 2B, wherein the front section is illustrated in its flat-out and folded state, respectively;
Figure 3A and Figure 3B are schematic top plan views of an exemplary embodiment of an absorbent article according to the invention wherein the first and second aspects are combined (front & rear pocket), wherein the elastic front and rear sections are illustrated in their flat-out and folded state, respectively;
Figure 4A is a partial top plan view of a preferred embodiment;
Figure 4B, Figure 4C and Figure 4D, Figure 4E, Figure 4F are schematic side views of figure 4A showing several options for where the hydrophilic surface (Aₚₕᵢ) can be present.
Figure 5A is a partial top plan view of an exemplary embodiment; and
Figure 5B and Figure 5C are schematic side views of the exemplary embodiment of figure 5A.
Figure 6A and Figure 6B illustrate perspective views of an exemplary embodiments.

### DESCRIPTION OF EMBODIMENTS

Generally applicable herein, the pocket may be arranged at the front of the article (e.g. as a pee pocket) or may be a arranged at the back of the article (e.g. as a poo pocket), the combination of having both pockets (pee & poo) is also possible. It is further understandable that the features described in the context of a front or rear guard pocket (e.g., a waist guard pocket) are equally applicable to another pocket as described herein. Depending on the position, the waist guard pocket may also be called a front or rear guard pocket. A feature described for the front pocket also applies for the back pocket and visa versa.

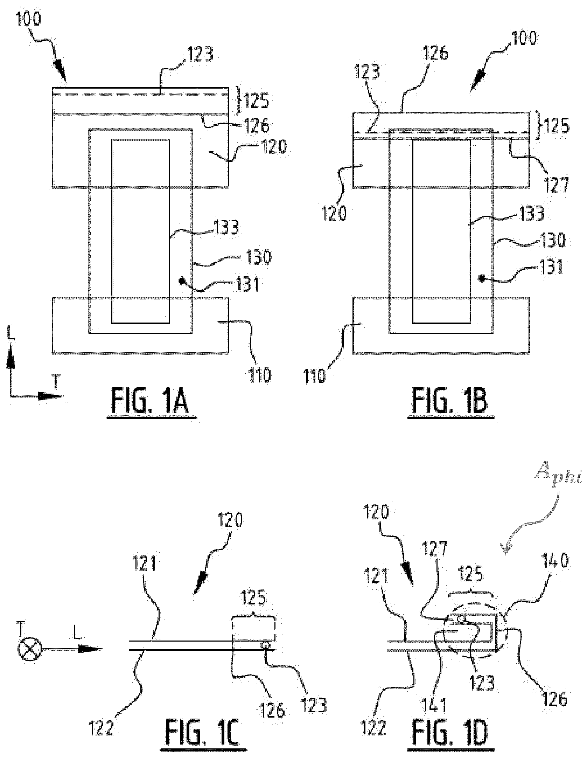

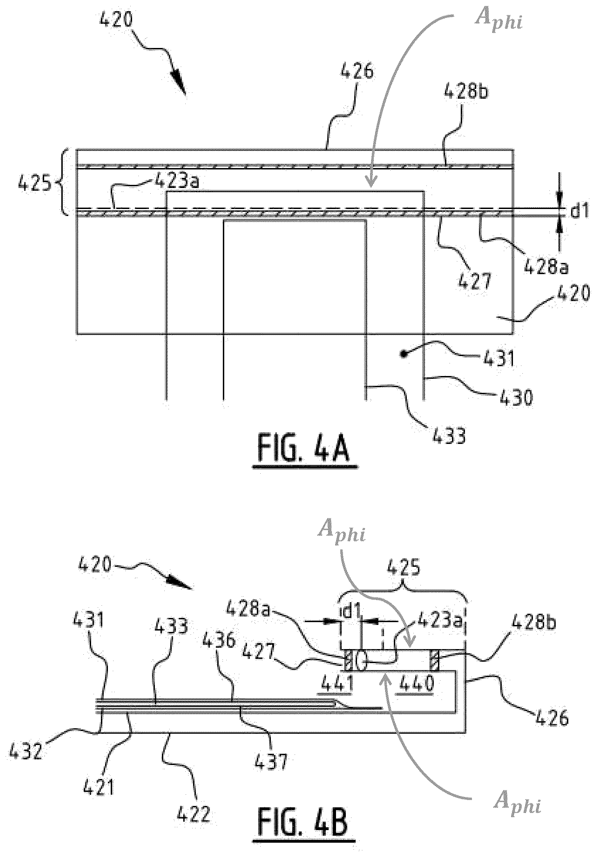

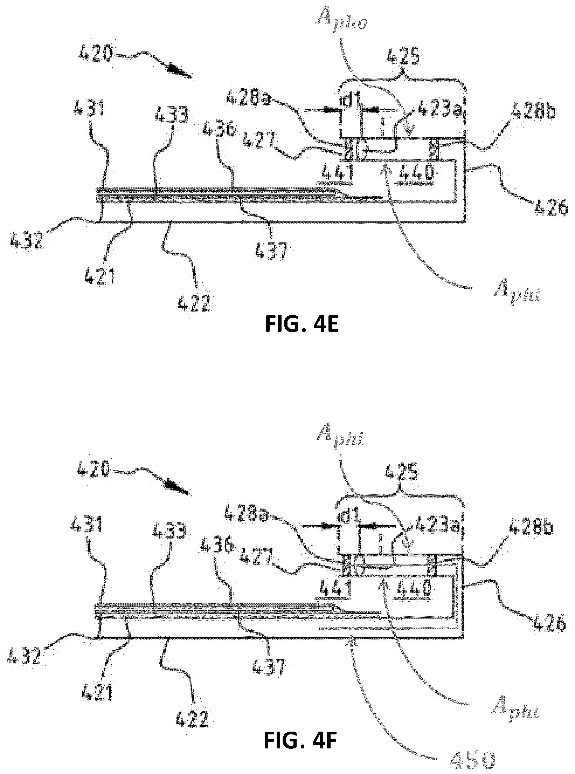

Figures 1A and 1B are schematic top views of an exemplary embodiment of an absorbent article 100 according to the first aspect of the invention. In figure 1A the elastic rear section 120 is shown in a flat-out state for illustrative purposes, and in figure 1B the elastic rear section 120 is shown in a folded state, which corresponds with the state of the absorbent article 100 when worn. Figures 1C and 1D are schematic side views in the transversal direction of the elastic rear section 120. Similarly figure 1C shows the elastic rear section 120 in the flat-out state for illustrative purposes, and figure 1D shows the elastic rear section 120 in the folded state. For reasons of clarity, figures 1C and 1D only show the elastic rear section 120. The absorbent body 130 which is partially supported by the elastic rear section 120 is not shown. A more complete view is presented in figure 4B. For the sake of completeness it is noted that elements in figures 1A and 1B are drawn as see-through elements.

However, it will be clear to the skilled person that in figure 1A the absorbent body 130 which comprises the absorbent core 133 is supported by the front elastic section 110 and rear elastic section 120, and that in figure 1B the rear folded portion 125 extends partially over the absorbent body 130. Figures 1A-1D illustrate an absorbent article 100, preferably baby pants, comprising an elastic front section 110, an elastic rear section 120, and an absorbent body 130 extending in the longitudinal direction L between the elastic front section 110 and the elastic rear section 120. The elastic rear section 120 comprises a laminate of an inner sheet 121 and an outer sheet 122. Between the inner sheet 121 and outer sheet 122 at least one elastic member 123 is provided. The absorbent body 130 comprises a liquid pervious topsheet 131, a liquid impervious backsheet 132, and an absorbent core 133 positioned between the topsheet 131 and backsheet 132. For reasons of clarity the backsheet 132 of the absorbent body 130 is not shown here. The elastic rear section 120 comprises a rear folded portion 125 where the laminate of the elastic rear section 120 is folded from a rear waistline 126 in the direction of the topsheet 131 of the absorbent body 130. In figures 1A and 1C the waistline 126 corresponds with an imaginary folding line at which the rear elastic section 120 is to be folded. In figures 1B and 1D, wherein the elastic rear section 120 is shown in its folded state, the waistline 126 is an actual waistline. The elastic member 123 is positioned within the rear folded portion 125 and is illustrated in a dashed line for illustrative purposes but it is clear to the skilled person that the elastic member 123 extends continuously in the transversal direction T. Additional elastic members may be provided in the rear folded portion 125 and/or in the remainder of the rear elastic section 120 other than the rear folded portion 125. This remainder is also referred to as rear unfolded portion and corresponds with the part of the elastic rear section 120 at the left-hand side of the indicated rear waistline 126 in figure 1C. The elastic member 123 and other elastic members that may be provided preferably are elastic strands which are at least partially covered with adhesive before being positioned within the laminate. The adhesive serves to keep the elastic members 123 in position and aids in keeping the laminate of the inner sheet 121 and outer sheet 122 together. The rear folded portion 125 extends from the rear waistline 126 to a free edge 127 of the rear folded portion 125 and thereby forms a rear guard pocket 140.

Generally, the front and/or rear folded portion as shown may comprises hydrophilic surface on the inside of the pocket and/or on the outside of the pocket.

Generally applicable herein (in particular applicable to all embodiments and aspects), the guard pocket comprises a hydrophilic surface (Aₚₕᵢ). The hydrophilic surface is indicated in the figures by reference "Aphi" .

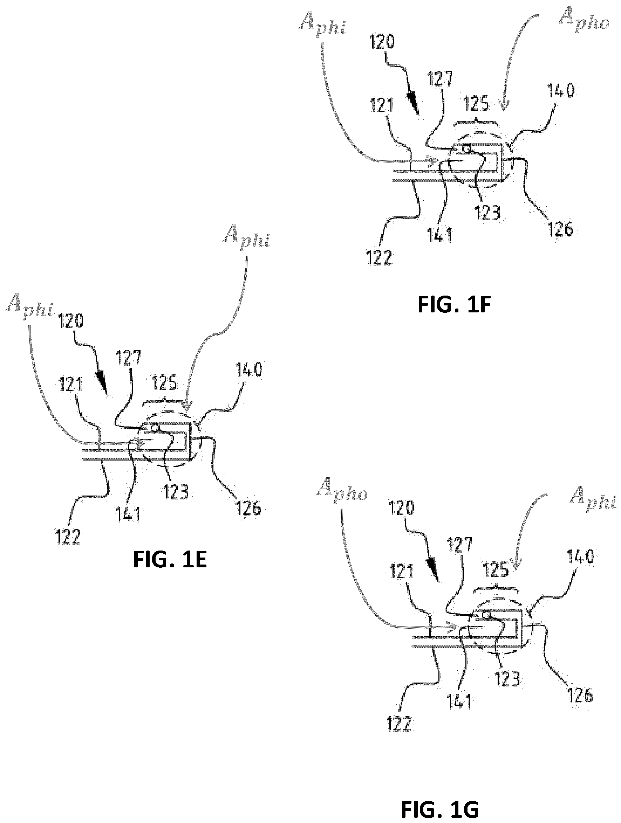

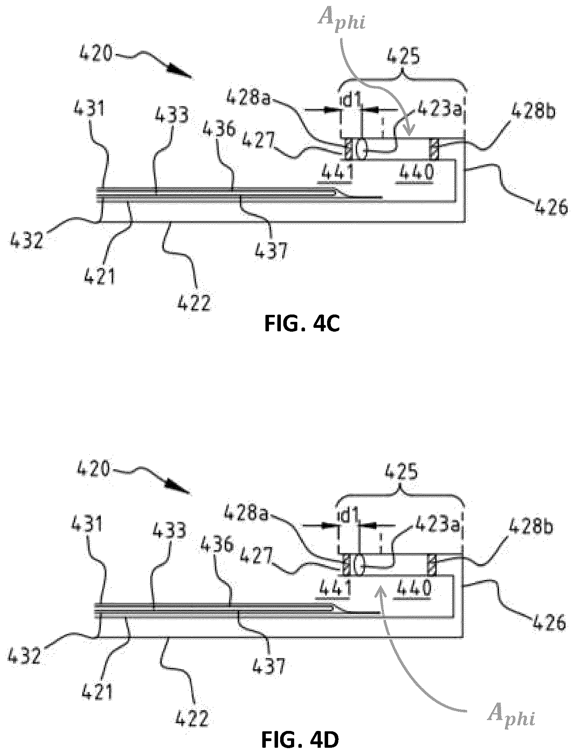

Hydrophilic surface may be facing towards the inside of the pocket (as shown in Figures 1D, 1E, 1F, 1F and figure 4D, 4E, 4D) and/or hydrophilic surface (Aₚₕᵢ) may be facing outwards (as shown in Figures 1D, 1G and Figure 4C, 4D). By having hydrophilic surface as described herein, comfort can be improved, more in particular, depending on where the hydrophilic surface is present:
- when facing outwards, comfort of the wearer may be improved due to increased softness;
- when facing inwards, comfort of the wearer may be improved due to improved containment of excretions or bodily exudates, namely the hydrophilic surface on the inside of the pocket may assist in capturing the excretions or bodily exudates.

In addition to the hydrophilic surface, a hydrophobic surface may be present, the hydrophobic surface is indicated in the figures by reference "Apho". By including a hydrophobic surface barrier effects may be further improved. For example, a hydrophobic barrier may be included between inner and outer sheet of the pocket, see e.g. Fig 4D showing a hydrophobic barrier 450 while the inner and outer sheets of the pocket are hydrophilic for improved comfort. The hydrophobic barrier 450 may be arranged as an intermediate barrier layer between the inner and outer sheets of the pocket (front and/or rear).

For example, as shown in figure 1G, a hydrophobic surface is used on the inside of the pocket. In figure 1F, a hydrophobic surface is present at the outside of the pocket.

The rear guard pocket has a pocket opening 141 defined between the free edge 127 of the rear folded portion 125 and the topsheet 131 of the absorbent body 130. The pocket opening 141 is situated close to an end portion of the absorbent core 133 and is configured for the intake of bodily exudates moving from the absorbent core 133 towards the rear waistline 126.

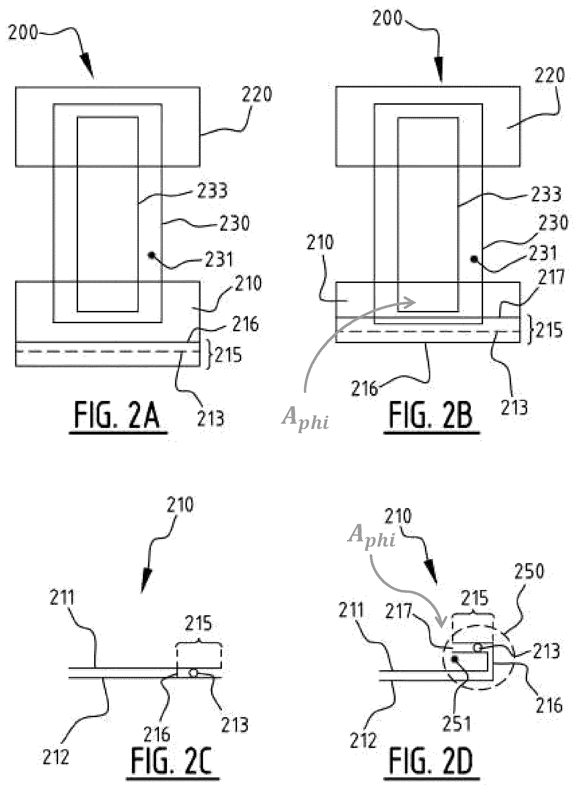

Figures 2A and 2B are schematic top views of an exemplary embodiment of an absorbent article 200 according to the second aspect of the invention. In figure 2A the elastic front section 210 is shown in a flat-out state for illustrative purposes, and in figure 2B the elastic front section 210 is shown in a folded state, which corresponds with the state of the absorbent article 200 when worn. Figures 2C and 2D are schematic side views in the transversal direction of the elastic front section 210. Similarly figure 2C shows the elastic front section 210 in the flat-out state for illustrative purposes, and figure 2D shows the elastic front section 210 in the folded state. For reasons of clarity, figures 2C and 2D only show the elastic front section 210. The absorbent body 230 which is partially supported by the elastic front section 210 is not shown. A more complete view is presented in figure 4B. For the sake of completeness it is noted that elements in figures 2A and 2B are drawn as see-through elements. However, it will be clear to the skilled person that in figure 2A the absorbent body 230 which comprises the absorbent core 233 is supported by the front elastic section 210 and rear elastic section 220, and that in figure 1B the front folded portion 215 extends partially over the absorbent body 230.

Figures 2A-2D illustrate an absorbent article 200, preferably baby pants, comprising an elastic front section 210, an elastic rear section 220, and an absorbent body 230 extending in the longitudinal direction L between the elastic front section 210 and the elastic rear section 220. The elastic front section 210 comprises a laminate of an inner sheet 211 and an outer sheet 212.

Generally applicable, the inner sheet and/or the outer sheet are chosen from a treated nonwoven as described herein. Preferably a treated nonwoven comprising polyethylene (PE), polypropylene (PP), polyester or polyethylene terephthalate (PET) or combinations thereof, wherein the nonwoven is treated to have a hydrophilic surface. Preferably, the nonwoven is treated with a hydrophilic surface coating, a hydrophilic spin finish or combinations thereof. Preferably, the nonwoven is treated with a hydrophilic spin finish. Generally applicable, the spin finish is preferred because it provides the hydrophilic character in an efficient and durable manner.

According to an embodiment, and generally applicable herein, the outer sheet of the pocket (front and/or rear) is a spunbond nonwoven and the inner sheet of said pocket is a Spunbond-Meltblown-Spunbond nonwoven. Advantageously, processing while ensuring comfort can be further increased, in particular in case at least one of the inner and outer sheet is treated to have hydrophilic surface, in particular treated with a hydrophilic spin finish.

As shown, between the inner sheet 211 and outer sheet 212 at least one elastic member 213 is provided. The absorbent body 230 comprises a liquid pervious topsheet 231, a liquid impervious backsheet 232, and an absorbent core 233 positioned between the topsheet 231 and backsheet 232. For reasons of clarity the backsheet 232 of the absorbent body 230 is not shown here. The elastic front section 210 comprises a front folded portion 215 where the laminate of the elastic front section 210 is folded from a front waistline 216 in the direction of the topsheet 231 of the absorbent body 230. In figures 2A and 2C the waistline 216 corresponds with an imaginary folding line at which the front elastic section 210 is to be folded. In figures 2B and 2D, wherein the elastic front section 210 is shown in its folded state, the waistline 216 is an actual waistline. The elastic member 213 is positioned within the front folded portion 215 and is illustrated in a dashed line for illustrative purposes but it is clear to the skilled person that the elastic member 213 extends continuously in the transversal direction T. Additional elastic members may be provided in the front folded portion 215 and/or in the remainder of the front elastic section 210 other than the front folded portion 215. This remainder is also referred to as front unfolded portion and corresponds with the part of the elastic front section 210 at the left-hand side of the indicated front waistline 216 in figure 2C. The elastic member 213 and other elastic members that may be provided preferably are elastic strands which are at least partially covered with adhesive before being positioned within the laminate. The adhesive serves to keep the elastic members 213 in position and aids in keeping the laminate of the inner sheet 221 and outer sheet 222 together. The front folded portion 215 extends from the front waistline 216 to a free edge 217 of the front folded portion 215 and thereby forms a front guard pocket 250. The front guard pocket 250 has a pocket opening 251 defined between the free edge 217 of the front folded portion 215 and the topsheet 231 of the absorbent body 230. The pocket opening 251 is situated close to an end portion of the absorbent core 233 and is configured for the intake of bodily exudates moving from the absorbent core 233 towards the front waistline 216.

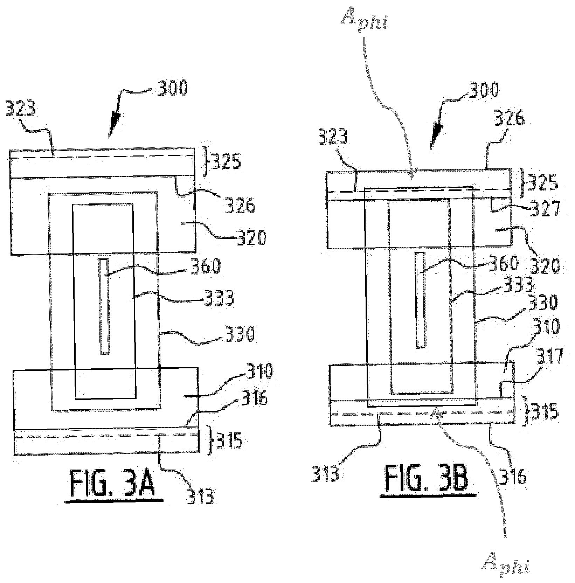

Figures 3A and 3B are schematic top views of an exemplary embodiment of an absorbent article 300 according wherein the first aspect and second aspect of the invention are combined. In figure 3A the elastic front section 310 and the elastic rear section 320 are shown in a flat-out state for illustrative purposes, and in figure 3B the elastic front section 310 and the elastic rear section 320 are shown in a folded state, which corresponds with the state of the absorbent article 300 when worn. For the elastic rear section 320 a similar description as given in view of elastic rear section 120 in figures 1A-1D applies, mutatis mutandis. A further detailed description is therefore omitted here for reasons of conciseness. For the elastic front section 310 a similar description as given in view of elastic front section 210 in figures 2A-2D applies, mutatis mutandis. A further detailed description is therefore omitted here for reasons of conciseness. In essence, the embodiment of figures 3A and 3B corresponds with an absorbent article 300, preferably baby pants, which combines the elastic rear section 120, 320 of the embodiment of figures 1A-1D, with the elastic front section 210, 310 of the embodiment of figures 2A-2D, with the additional feature that the absorbent core 333 is provided with a channel 360. In the channel 360, or multiple channels 360 (not shown) less absorbent material per surface area is present as compared to an area of the absorbent core 333 around the channel 360. Preferably substantially no absorbent material is present in the at least one channel 360. It is clear that the provision of one or more channels 360 is not necessarily and exclusively linked to the embodiment of figures 3A-3B, and that one or more channels 360 may be provided in the embodiments of figures 1A-1D and figures 2A-2D as well. For the sake of conciseness a further detailed description of possible absorbent core configurations with one or more channels 360 are omitted here. Absorbent articles comprising an absorbent core with one or more channels between a topsheet and a backsheet are well known. Examples of such absorbent articles are described for example in patents EP 3 403 630 B1, EP 3 403 632 B1 and patent application EP 3 403 631 A1 in the name of the applicant, which are incorporated herein by reference. The applicant has found that it is especially beneficial in embodiments of absorbent articles with elastic front and/or rear sections with corresponding front and/or rear guard pockets to have an absorbent core with one or more channels wherein the majority of the channel(s) extends substantially in the longitudinal direction, i.e. towards the front and/or rear guard pocket.

Figure 4A is a top plan view of an elastic rear section 420 of an absorbent article according to a preferred embodiment. Figure 4B is a schematic side view of the elastic rear section 420 as illustrated in figure 4A. Figures 4A and 4B show an elastic rear section 420 comprising a laminate of an inner sheet 421, an outer sheet 422, and at least one elastic member 423 positioned between the inner sheet 421 and the outer sheet 422. The elastic rear section 420 comprises a rear folded portion 425 wherein the laminate is folded from a rear waistline 426 of the elastic rear section towards the topsheet 431 of the absorbent body 430. The rear folded portion comprises hydrophilic surface at the inside and/or at the outside, as is better visible in figure 4B shown an example of hydrophilic surface at the inside and the outside. It is also possible that only the inside or the outside is hydrophilic while the other side is hydrophobic (as illustrated by the possible options shown by fig. 1E, 1F, 1G, which is applicable mutatis mutandis, and as shown by the possible options illustrated by fig. 4B, 4C, 4D). The rear folded portion 425 extends from the rear waistline 426 to a free edge 427 of the rear folded portion 425 to form a rear guard pocket 440 having a pocket opening 441 defined between the free edge 427 of the rear folded portion 425 and the topsheet 431 of the absorbent body 430. The rear folded portion 425 comprises a first elastic member 423a in proximity of and substantially parallel with the free edge 427. The maximum distance d1 between the first elastic member 423a and the free edge 427 should be as small as possible and less than 15mm. Preferably the distance d1 is less than 12mm, more preferably less than 9mm, and most preferably less than 6mm. In close proximity of the first elastic member 423a, and preferably adjacent thereto, there is provided a first attachment zone 128a, in the form of a strip of adhesive, wherein the inner sheet 421 and outer sheet 422 are attached to each other. The first attachment zone 428a is positioned between the free edge 427 and the first elastic member 423a. In the illustrated embodiment the rear folded portion 425 comprises a second attachment zone 428b wherein the inner sheet 421 and outer sheet 422 are attached to each other. The second attachment zone 128b is positioned in between the first elastic member 423a and a rear baseline of the rear guard pocket. The baseline corresponds with the position from whereon the rear folded portion 425 starts to open up towards the free edge 427, i.e. from where there is a distance between the inner sheet 421 of the rear folded portion 425 and the inner sheet 421 of the unfolded portion of the elastic rear section 420. In the illustrated embodiment the baseline of the rear guard pocket is the rear waistline 426. In a further embodiment the rear folded portion 425 may comprise at least one second elastic member (not shown) positioned between the first elastic member 423a and the second attachment zone 428b, preferably in close proximity of the second attachment zone 428b. On the other hand, it is clear that other embodiment exist wherein the second attachment zone 428b is not provided. Figure 4B further illustrates that the absorbent body 430 is provided partially on top of the inner sheet 421 of the elastic rear section 420. The absorbent body 430 comprises a liquid pervious topsheet 431, a liquid impervious backsheet 432, and an absorbent core 433 positioned between the topsheet 431 and backsheet 432. The absorbent core 433 comprises a bottom core wrap 437, a top core wrap 436 and absorbent material therebetween.

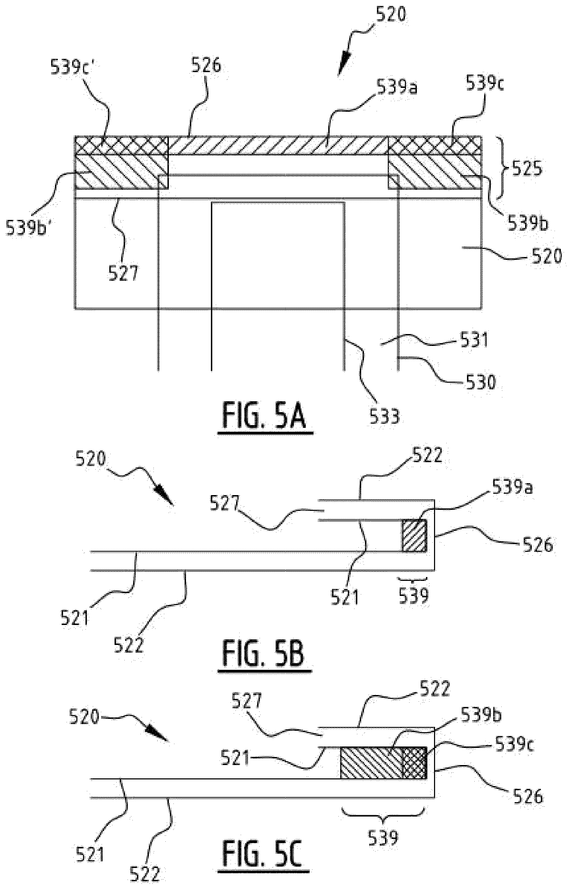

Figure 5A is a top plan view of an elastic rear section 520 of an absorbent article according to a preferred embodiment. Figures 5B and 5C are schematic side views of the elastic rear section 520 as illustrated in figure 5A. More in particular figure 5B shows a side view of a cross section taken along a longitudinal center line, and figure 5C shows a side view of a cross section taken along a longitudinal side line. It is noted that figures 5A, 5B and 5C are intended to illustrate an embodiment of a rear elastic section 520 having a closed edge portion 539, and therefore these figures do not show any elastic members or attachment zones in the laminate of the elastic rear section 520 for the sake of clarity. At the closed edge portion 539 the inner sheet 521 of the rear folded portion 525 is attached to or connected with the inner sheet 521 of the unfolded portion of the elastic rear section. The closed edge portion 539 provides additional stability to the guard pocket formed by the rear folded portion 525, because the rear folded portion 525 is partially adhered to unfolded portion. The closed edge portion 539 is configured such that there is still a sufficiently large part of the rear folded portion 525 that opens up in an adequate manner to receive bodily exudates. The rear folded portion may comprise the hydrophilic surface at the inside and/or at the outside of the pocket (as shown by

Figure 5B). More in particular, the illustrated closed edge portion 539 comprises three edge subportions 539a, 539b and 539b'. Subportion 539a is substantially strip shaped and extends parallel to the rear waistline 526 and preferably adjacent thereto. Subportions 539b and 539b' are substantially rectangular and are positioned at each corner of the elastic rear section 520 which borders the rear waistline. Subportions 539a and subportions 539b, 539b' partially overlap in overlap sections 539c, 539c'. The closed edge portion 539 is formed by applying adhesive on the inner sheet 521 in accordance with the to be formed closed edge portion 539 prior to folding the elastic rear section 520. In the illustrated embodiment the baseline of the rear guard pocket is determined by the closed edge portion 539, especially by subportion 539a. It is clear that each of the indicated subportions 539a, 539b, 539b' can be provided separately, or in any combination.

Although figures 4A-4B and 5A-5C and the corresponding description is aimed at specific embodiments of a rear elastic section, the illustrated and described features, in particular in relation to the hydrophilic surface, preferably provided by a treated nonwoven described herein,apply in a similar manner to embodiments of a front elastic section, mutatis mutandis. Front and rear elastic sections or panels of absorbent articles such as baby diapers or baby pants are often made from similar materials but might have slightly different dimensions and configurations. For example, a rear elastic section according to an embodiment of the invention might have a width (transversal direction) of about 405 mm and a length (longitudinal direction) of about 170 mm in the folded state, wherein the rear folded portion has a length of about 60 mm, while a front elastic section according to an embodiment of the invention might have a width (transversal direction) of about 405 mm and a length (longitudinal direction) of about 130 mm in the folded state, wherein the front folded portion has a length of about 55 mm. It is clear that these dimensions may vary depending on the size of the absorbent article.

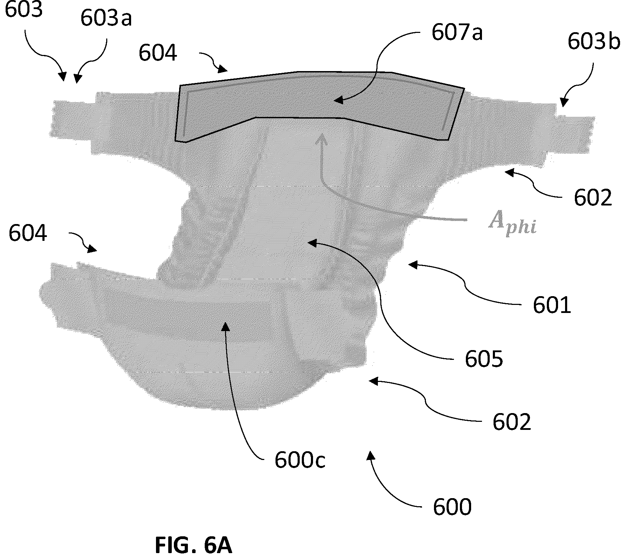

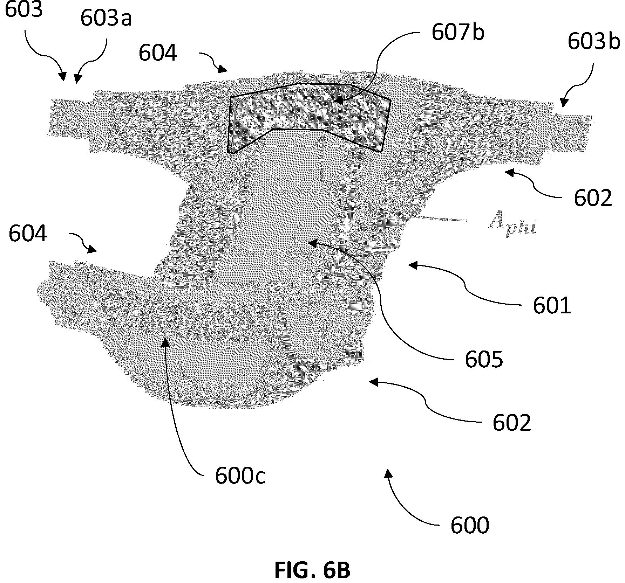

Figure 6A and figure 6B show examples of an absorbent article 600 comprising an absorbent body 601 extending in a longitudinal direction between a front section 602 and a rear section 603 of a waistband portion 604, wherein the absorbent body 601 comprises a liquid pervious topsheet 605, a liquid impervious backsheet, and an absorbent core positioned between the topsheet and backsheet.

As shown, the article comprises fastening means 603 or fastening system 603 for fastening the rear section a waistband portion to the front section the waistband portion. Any suitable fastening means or system 603 for a diaper can be used, which is preferable designed to provide a secure, adjustable, and comfortable fit while minimizing the risk of leaks and skin irritation can be used. The fastening means may for example include a pair of fastening tabs 60a3, 603b, each affixed to opposing lateral portions of a waistband portion . The fastening means may include a hook-and-loop fastener system, for example wherein each tab includes a hook component that engages with a looped landing zone 600c positioned on a front waistband portion.

Figure 6A and figure 6B further show that the absorbent article is provided with a waist guard pocket 607a, 607b which may be a front guard pocket (not shown in the perspective view) or rear guard pocket (as shown). Understandable, the combination of two waist guard pockets, e.g. front guard pocket and rear guard pocket is also possible as explained earlier.

The waist guard pocket comprises a hydrophilic surface (Aₚₕᵢ), either on the inside or on the outside of the pocket or both. Understandably, the hydrophilic surface(s) may be included into the embodiments of Figure 6A and figure 6B as described earlier herein.

Figure 6A shows that waist guard pocket 607a is provided by folding the upper edge of the waistband.

Figure 6B shows that is also possible that the waist guard pocket 607a is provided as a patch (as shown by figure 6B), wherein a perimeter of the patch is sealed via any suitable technique.

Further preferred details with respect to the provision of the pocket as a patch are described in EP application nr 25164573.5, in the name of the applicant and which is incorporated herein by reference, in particular regarding the inclusion of the "separate patch". Further details with respect to the provision of the front and/or rear guard pocket as a patch are described in NL application nr 2039889 in the name of the applicant and which is incorporated herein by reference, in particular regarding the inclusion of the "separate patch". It will be clear to the skilled person that in addition to the illustrated and described elastic members, additional elastic members may be present in the elastic front and/or rear sections, in the folded and/or unfolded portions. For example, an absorbent article having the exemplary dimensions as described above might have a total of between 1 and 6 elastic members in the front or rear folded portions and a total of between 8 and 24 elastic members in the front or rear unfolded portions.

### Glossary

As used in the present application, the following terms have the following meanings:
"A", "an", and "the" as used herein refers to both singular and plural referents unless the context clearly dictates otherwise. By way of example, "an edge barrier" refers to one or more than one edge barrier.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.

"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.

"Absorbent core" as used herein refers to a three-dimensional part of the absorbent structure, comprising liquid-absorbing material, useful to permanently absorb and/or retain bodily exudates. "Absorbent component" as used herein refers to a structural constituent of an absorbent article, e.g., a piece of an absorbent core, such as one of multiple pieces in a multi-piece absorbent core. "Absorbent element" as used herein refers to a part of a functional constituent of an absorbent structure, e.g., an acquisition layer, a dispersion layer, core layer or a release structure formed of a material or materials having particular liquid handling characteristics suitable for the specific function.

"Absorbent fibrous polymer material" as used herein refers to an absorbent polymer material which is in threadlike from such as fibers, filaments, and the like so as to be less flowable in the dry state than particulates.

"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.

"Absorbent polymer material", "absorbent gelling material", "AGM", "superabsorbent", "superabsorbent material", "superabsorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).

"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artifacts).

"Absorbent particulate polymer material" as used herein refers to an absorbent polymer material which is in particulate form such as powders, granules, flakes and the like so as to be flowable in the dry state.

"Absorption" as used herein refers to the process by which a liquid is taken up within a material.

"Absorption rate" as used herein refers to the rate of absorption of liquid, i.e. the amount of liquid which is absorbed per unit of time, typically by an absorbent component, element and/or absorbent layer of the absorbent article, structure and/or core.

"Acquisition layer", "acquisition region", "acquisition surface" or "acquisition material" and the like as used herein refer to the layer overlying the absorbent core having a faster liquid uptake and/or distribution capability.

"Absorbency" is the ability of a material to take up fluids by various means including capillary, osmotic, solvent, chemical and/or other action.

"Adult incontinence garment" as used herein refers to absorbent articles intended to be worn by incontinent adults, for absorbing and containing bodily exudates.

"Adhesion" as used herein refers to the force that holds different materials together at their interface.

"Adhesive" as used herein refers to a material, which may or may not be flowable in solution or when heated, that is used to bond materials together.

"Adsorption" as used herein refers to the process by which a liquid is taken up by the surface of a material.

"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".

"Apparent density", "density" as used herein refers to the basis weight of the sample divided by the caliper with appropriate unit conversions incorporated therein. Apparent density used herein has the unit g/cm3.

"Attach", "attached" and "attachment" as used herein are synonymous with their counterparts of the terms "fasten", "affix", "secure", "bind", "join" and "link".

"Baby diaper" as used herein refers to absorbent articles intended to be worn by children, for absorbing and containing bodily exudates which the user draws up between the legs and fastens about the waist of the wearer.

"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.

"Back region" as used herein refers to the portion of an absorbent article or part thereof that is intended to be positioned proximate the back of a wearer.

"Backing" as used herein refers to a web or other material that supports and reinforces the back of a product.

"Basis weight" is the weight per unit area of a sample reported in grams per square meter, g/m2 or gsm.

"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.

"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.

"Bond strength" as used herein refers to the amount of adhesion between bonded surfaces. It is a measure of the stress required to separate a layer of material from the base to which it is bonded. "Capillary action", "capillarity", or "capillary motion" and the like as used herein are used to refer to the phenomena of the flow of liquid through porous media.

"Chassis" as used herein refers to a foundational constituent of an absorbent article upon which the remainder of the structure of the article is built up or overlaid, e.g., in a diaper, the structural elements

that give the diaper the form of briefs or pants when configured for wearing, such as a backsheet, a topsheet, or a combination of a topsheet and a backsheet.

"Cellulose fibers" as used herein refers to naturally occurring fibers based on cellulose, such as, for example cotton, linen, etc.; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers.

"Cluster" or the like as used herein refers to an agglomeration of particles and/or fibers.

"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc.), altering the chemical structure of the fibers themselves (e.g. by cross-linking polymer chains, etc.) and the like.

"Cohesion" as used herein refers to the resistance of similar materials to be separated from each other.

"Compartment" as used herein refers to chambers, cavities, pockets and the like.

"Comprise," "comprising," and "comprises" and "comprised of" as used herein are synonymous with "include", "including", "includes" or "contain", "containing", "contains" and are inclusive or openended terms that specify the presence of what follows e.g. a component and do not exclude or preclude the presence of additional, non-recited components, features, elements, members, steps, known in the art or disclosed therein.

"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.

"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.

"Cross direction (CD)", "lateral" or "transverse" and the like as used herein are used interchangeably and refer to a direction which is orthogonal to the longitudinal direction and includes directions within ±45° of the transversal direction.

"Curing" as used herein refers to a process by which resins, binders or plastics are set into or onto fabrics, usually by heating, to cause them to stay in place; the setting may occur by removing solvent or by cross-linking so as to make them in soluble.

"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.

"Dispersion layer", "dispersion region", "dispersion surface" or "dispersion material" and the like as used herein refer to the layer overlying the absorbent core having a faster liquid uptake and dispersion capability.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".

"Dry strength" as used herein refers to the strength of a joint determined in dry state conditions, immediately after drying under specified conditions or after a period of conditioning in the standard laboratory atmosphere.

"Essentially cellulose free", "substantially fluffless" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.

"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.

"Fabric" as used herein refers to a sheet structure made from fibers, filaments and/or yarns. "Feminine hygiene garments" as used herein refer to absorbent hygiene articles intended to be worn by woman, for absorbing and containing body exudates.

"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.

"Fluff pulp" or "Pulp fluff" as used herein refers to wood pulp specially prepared to be drylaid. The fibers can be either natural or synthetic or a combination thereof.

"Front region" as used herein refers to the portion of an absorbent article or part thereof that is intended to be positioned proximate the front of a wearer.

"Garment facing layer" as used herein refers to elements of the chassis that form the outer surface of the absorbent article, such as the backsheet, the side panels, the waist fasteners, and the like, when such elements are present.

"Heat activated adhesive" as used herein refers to a dry adhesive that is rendered tacky or fluid by application of heat or heat and pressure to the assembly.

"Heat sealing adhesive" as used herein refers to a thermoplastic adhesive which is melted between the adherent surfaces by heat application to one or both of the adjacent adherent surfaces.

"High loft" as used herein refers to general term of low density, thick or bulky fabrics.

"Hot-melt adhesive" as used herein refers to a solid material that melts quickly upon heating, then sets to a firm bond upon cooling; used for almost instantaneous bonding.

"Hydrophilic" as used herein refers to having an affinity for being wetted by water or for absorbing water.

Generally herein, "hydrophilic" or hydrophilicity (or hydrophobicity) may be determined via ISO-9073-11:2002(E). See first edition 2002-11-01 , textiles - test methods for nonwovens - part 11.

"Hydrophobic" as used herein refers to lacking the affinity for being wetted by water or for absorbing water.

"Immobilization layer" as used herein refers to a layer able to be applied to the absorbent polymer material or absorbent polymer material area with the intent to gather, bond and/or immobilize absorbent material and/or absorbent layer.

"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.

"Knitting" as used herein refers to the technique for interlocking loops of fibers with needles or similar devices.

"Layer" refers to identifiable components of the absorbent article, and any part referred to as a "layer" may actually comprise a laminate or combination of several sheets or webs of the requisite type of materials. As used herein, the term "layer" includes the terms "layers" and "layered."

"Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration .

"Machine direction (MD)", "longitudinal" and the like as used herein are used interchangeably and refer to a direction running parallel to the maximum linear dimension of the structure and includes directions within ±45° of the longitudinal direction.

"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

"Mass flow" as used herein refers to the flow of a liquid from one absorbent element or component to another absorbent element or component by channel flow action.

"Mechanical bonding" as used herein refers to a method of bonding fibers by entangling them. This can be achieved by needling, stitching with fibers or by the use of high-pressure air or water jets and the like.

"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).

"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).

"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.

"Rear" as used herein refers to the portion of an absorbent article or part thereof that is intended to be positioned proximate the back of the wearer.

"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.

"Resin" as used herein refers to a solid or semisolid polymeric material.

"Thermobonding" as used herein refers to a method of bonding fibers by the use of heat and/or high-pressure.

"Thermoplastic" as used herein refers to polymeric materials that have a melting temperature and can flow or be formed into desired shapes on the application of heat at or below the melting point.

"Ultrasonic" as used herein refers to the use of high frequency sound to generate localized heat through vibration thereby causing thermoplastic fibers to bond to one another.

"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.

"Wearer facing layer" as used herein refers to elements of the chassis that form the inner surface of the absorbent article, such as the topsheet, the leg cuffs, and the side panels, etc., when such elements are present.

"Weaving" as used herein refers to the process of interlacing two or more sets of yarns at right angles to form a fabric; a web of fibers produced by weaving is herein referred to as a "woven".

"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.

"Wet burst strength" is a measure of a layer's ability to absorb energy, when wet and subjected to deformation normal to the plane of the web.

"Wet strength" as used herein refers to the strength of a joint determined immediately after removal from a liquid in which it has been immersed under specified conditions of time, temperature and pressure. The term is commonly used in the art to designate strength after immersion in water.

"Wetlaying" as used herein refers to the forming a web from an aqueous dispersion of fibers by applying modified paper making techniques; a web of fibers produced by wetlaying is herein referred to as a "wetlaid".

"Wood pulp" as used herein refers to cellulosic fibers used to make viscose rayon, paper and the absorbent cores of products such as feminine hygiene garments, baby diapers and pants and adult incontinence garments.

"X-y dimension" as used herein refers to the plane orthogonal to the thickness of the article, structure or element. The x- and y-dimensions correspond generally to the width and length, respectively, of the article, structure or element.

"Z-dimension" as used herein refers to the dimension orthogonal to the length and width of the article, structure or element. The z-dimension corresponds generally to the thickness of the article, structure or element.

## Claims

1. An absorbent article comprising an elastic front section, an elastic rear section, and an absorbent body extending in a longitudinal direction between the elastic front section and the elastic rear section,
wherein the elastic front section and the elastic rear section each comprise a laminate comprising an inner sheet, an outer sheet, and at least one elastic member positioned between the inner sheet and the outer sheet;
wherein the absorbent body comprises a liquid pervious topsheet, a liquid impervious backsheet, and an absorbent core positioned between the topsheet and backsheet;
the absorbent article comprising
a rear guard pocket extending transversely across the elastic rear section, the rear guard pocket having a rear guard pocket opening for receiving excretions; wherein the rear guard pocket comprises a hydrophilic surface (Aₚₕᵢ).

2. The absorbent article according to any one of the preceding claims,
wherein the rear guard pocket comprises an inside portion and an outside portion;
wherein the hydrophilic surface (Aₚₕᵢ) is present on the inside portion and/or the outside portion rear guard pocket.

3. The absorbent article of the former claim,
wherein the hydrophilic surface (Aₚₕᵢ) is present on the an inside portion of the rear guard pocket.

4. The absorbent article of any of the former two claims,
wherein the hydrophilic surface (Aₚₕᵢ) is present on the outside portion of the rear guard pocket.

5. An absorbent article comprising an elastic front section, an elastic rear section, and an absorbent body extending in a longitudinal direction between the elastic front section and the elastic rear section,
wherein the elastic front section and the elastic rear section each comprise a laminate comprising an inner sheet, an outer sheet, and at least one elastic member positioned between the inner sheet and the outer sheet;
wherein the absorbent body comprises a liquid pervious topsheet, a liquid impervious backsheet, and an absorbent core positioned between the topsheet and backsheet;
the absorbent article comprising
a front guard pocket extending transversely across the elastic front section, the front guard pocket having a front guard pocket opening for receiving excretions wherein the front guard pocket comprises a hydrophilic surface (Aₚₕᵢ).

6. The absorbent article according to claim 5,
wherein the front guard pocket comprises an inside portion and an outside portion;
wherein the hydrophilic surface (Aₚₕᵢ) is present on the inside portion and/or the outside portion front guard pocket.

7. The absorbent article of any of claims 6,
wherein the hydrophilic surface (Aₚₕᵢ) is present on the inside portion of the front guard pocket.

8. The absorbent article of any of claims 6 - 7,
wherein the hydrophilic surface (Aₚₕᵢ) is present on the outside portion of the front guard pocket.

9. An absorbent article comprising an absorbent body extending in a longitudinal direction between a front section and a rear section of the waistband portion,
wherein the article comprises fastening means, such as a pair of fastening tabs, for fastening the rear section of the waistband portion to the front section the waistband portion,
wherein the absorbent body comprises a liquid pervious topsheet, a liquid impervious backsheet, and an absorbent core positioned between the topsheet and backsheet;
the absorbent article comprising
at least one waist guard pocket, such as a front and/or a rear guard pocket, extending transversely across the waistband portion, the waist guard pocket having a pocket opening for receiving excretions;
wherein the waist guard pocket comprises a hydrophilic surface (Aₚₕᵢ).

10. The absorbent article according to claim 5,
wherein the waist guard pocket comprises an inside portion and an outside portion;
wherein the hydrophilic surface (Aₚₕᵢ) is present on the inside portion and/or the outside portion waist guard pocket.

11. The absorbent article according to any one of the preceding claims,
wherein the hydrophilic surface (Aₚₕᵢ) is present as surface of a nonwoven, preferably a nonwoven chosen from spunbond, meltblown, spunlace or spunbond-meltblown-spunbond.

12. The absorbent article according to any of the previous claims,
wherein the hydrophilic surface (Aₚₕᵢ) is present as a surface of a nonwoven arranged as an inner sheet of the pocket; and/or
wherein the hydrophilic surface (Aₚₕᵢ) is present as a surface of a nonwoven arranged as an outer sheet of the pocket.

13. The absorbent according to any one of the preceding claims,
wherein the hydrophilic surface (Aₚₕᵢ) is present as a surface of a nonwoven chosen from a treated nonwoven; or
wherein the hydrophilic surface (Aₚₕᵢ) is a surface of a nonwoven
which substantially consist of a hydrophilic material(s); or
wherein the hydrophilic surface (Aₚₕᵢ) is a surface of a hydrophilic nonwoven
chosen from a nonwoven comprising a mixture of hydrophobic polymers blended or grafted with hydrophilic additives.

14. The absorbent according to any one of the preceding claims,
wherein the hydrophilic surface (Aₚₕᵢ) is provided by a treatment chosen from a hydrophilic surface coating, a hydrophilic spin finish or combinations thereof.

15. The absorbent according to any one of the preceding claims,
wherein the pocket comprises a hydrophilic spin finish which provides the hydrophilic surface (Aₚₕᵢ).
